# EUROPEAN PATENT SPECIFICATION

(11) **EP 1 539 202 B1**
(45) Date of publication and mention of the grant of the patent: **21.07.2010**
(21) Application number: 03770255.2
(22) Date of filing: 21.08.2003
(51) Int. Cl.: A61K 31/00, A61K 35/00, A61K 36/00

(54) **Compositions based on plant extracts for the treatment of cancer**
Zusammensetzungen basierend auf Planzenextrakten zur Behandlung von Krebs
Compositions à base d'extraits d'herbes pour le traitement du cancer

(30) Priority: 23.08.2002 US 227006
(43) Date of publication of application: 15.06.2005
(73) Proprietor: Sterogene Bioseparations, Inc., Carlsbad, CA 92088 (US)
(72) Inventor: GRANDICS, Peter, Carlsbad, CA 92088 (US)
(74) Representative: Ritthaler, Wolfgang
(86) International application number: PCT/US2003/026765
(87) International publication number: WO 2004/017984

(56) References cited:
- GB-A- 864 740
- GRANDICS PETER: "Cancer: a single disease with a multitude of manifestions?" JOURNAL OF CARCINOGENESIS, [Online] vol. 2, no. 9, 18 November 2003 (2003-11-18), XP002456729 Retrieved from the Internet: URL:http://www.carcinogenesis.com/content/ pdf/1477-3163-2-9.pdf>
- MUHLHOLLAND, J.: SOC.SC AND MED., vol. 148, 1980, pages 181-184, XP009091316
- DATABASE WPI Week 198920 Derwent Publications Ltd., London, GB; AN 1989-147375 XP002446734 & JP 01 090129 A (KODAMA KK) 6 April 1989 (1989-04-06)
- ANCIENT SCIENCE OF LIFE, 1983, pages 161-167, XP009091347
- THAS J J: "Preliminary pharmacological findings on Ceropegia juncea (Abstract)" PLANTA MEDICA, THIEME, STUTTGART, DE, vol. 39, 1980, pages 242-242, XP009091346 ISSN: 0032-0943
- DATABASE WPI Week 199931 Derwent Publications Ltd., London, GB; AN 1999-367092 XP002457498 & JP 11 140102 A (AOMORI KEN) 25 May 1999 (1999-05-25)

## Description

The present invention relates to a therapeutic composition in accordance with claim 1.

### BACKGROUND OF THE INVENTION

*General Background and State of the Art:* It is a widely accepted view that cancer can start in just one of the body's billions of cells. Cancer could be triggered by a variety of factors. Our current thinking is that radiation, toxic chemicals, viruses or other infectious agents may induce an error in the transcription of the cell's genetic information. The cells then divide to form abnormal cells, without normal genetic controls. The immune system of the body then fails to respond property by not destroying the aberrant cells. The aberrant (cancerous) cells lose their normal controls of cell division and continue to proliferate. This leads to the formation of a growing mass or tumor expanding into healthy tissues. The cancerous cells compete with normal cells for nutrition. Also, the cancerous cells may migrate into the bloodstream or the lymphatic system that is the primary cause of the formation of a metastasis.

It is currently believed that cancer could be reversed if the altered genetic message of the cell could be corrected. However, such a method up to this date has not been developed. Our current mainstream treatment methods focus exclusively on the tumor and equate cancer with the cancerous lesion(s) appearing in these patients. This way cancer is considered a localized phenomenon that may lead to an incomplete definition of this disease.

The mainstream treatment modalities for cancer are sometimes described as the cut, bum and poison therapies. As the cancerous lesion is equated with cancer, its surgical removal, whenever is possible, is considered indispensable. This is done despite the evidence that surgery fails to correct the underlying cause of cancer and may actually cause the spreading of cancer. Residual lesions are treated with radiation and chemotherapy, both of which produce severe, sometimes lethal side effects. These treatments are immunosuppressive and can pave the way to secondary infections, an important cause of mortality following chemotherapy.

Toxicity to the kidneys, bone marrow and the nervous system may produce lasting complications even if a remission is achieved. It is also established that such therapies can actually cause secondary tumors. Regardless of the practice of these cancer treatments, two-thirds of all cancer patients eventually die of the disease. Moreover, many of the malignant tumors are resistant to these conventional treatments.

A safe and effective cancer treatment has been the goal of scientists for many decades. Such a technique must be selective in destroying the cancer cells without irreversibly damaging normal cells. It is well established that cancer is continually produced in the human body but is kept in check by the immune system. Only when the immune system is weakened can cancer establish itself. Therefore, it would be desirable to develop methods that restore the healing ability of the body so cancer would be eliminated naturally by the immune system.

### INVENTION SUMMARY

Pursuant to this invention a composition described to treat cancer. In an illustrative embodiment, an inoperable, malignant lung cancer was treated with a therapeutic composition comprised of a mixture of natural plant derived substances and minerals.

Methods disclosed in the present application are particularly suited to the treatment of all types of malignancies in the body. However, In one alternative, they are more particularly suited to the treatment of malignancies of the lymphoid system.

### BRIEF DESCRIPTION OF THE DRAWINGS

The following invention will become better understood with reference to the specification, appended claims, and accompanying drawing, where:

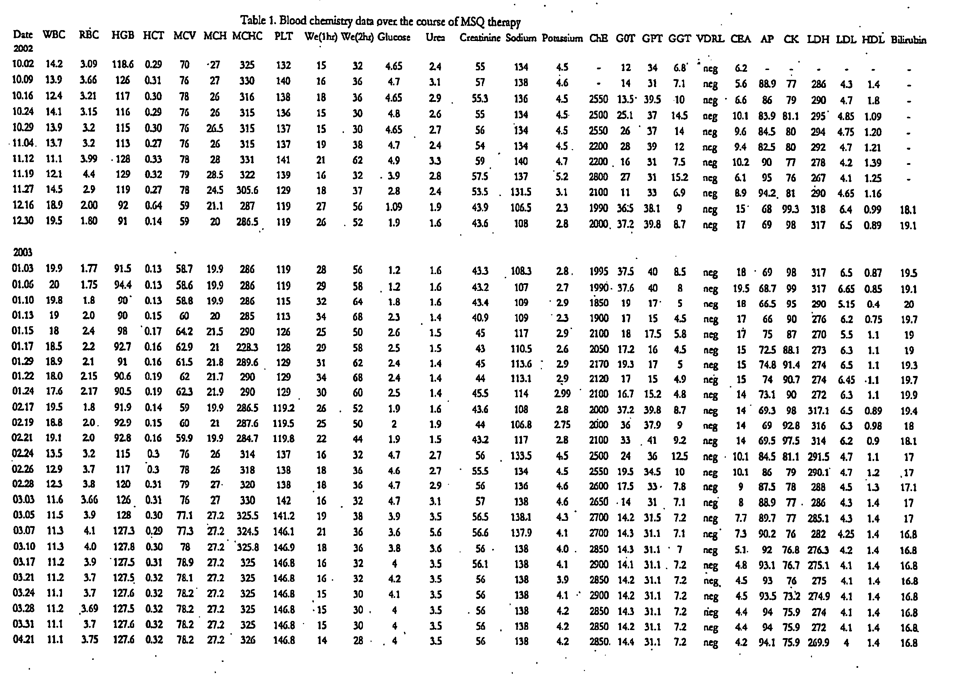

Figure 1 is a table showing the results of blood work over time for the patient of Example 3.

### DETAILED DESCRIPTION OF THE PREFERRED EMBODIMENTS

Interest in alternative therapies is increasing as dissatisfaction with traditional therapies grows. The absence of markedly improved treatments despite decades of research, the toxicity of chemotherapy and the lack of significant improvement in cure rates for the major cancers contribute to the dissatisfaction (Cassileth et al. (1991) New England Journal of Medicine 3249 (17) 1180-1185). This led to an increasing interest even within the traditional medical community for alternative cancer treatments.

The present invention describes an alternative therapy for cancer in accordance with a therapeutic composition of claim 1. We offer a new theory for the development of this disease that describes cancer as an endocrine disease. More specifically, we hypothesize that cancer manifests as a result of the malfunctioning of the pituitary gland that resides at the apex of the endocrine system. Pituitary hormones regulate the functions of other glands, e.g., the thyroid, the adrenals or the pancreas. A dysfunctional pituitary gland manifests in the weakening of the immune system and eventually in its inability to eliminate cancerous cells.

This hypothesis proposes that cancers arise from a single cancer "progenitor" cell that gives rise to all known forms of cancer. This cell is part of the working mechanism of the immune system and normally resides in the sinus cavity from which it is mobilized as needed. These cells are eliminated by specific activated lymphocytes after they complete their tasks. When the elimination of these cells is unsuccessful due to weakened thyroid, adrenal, and pancreatic activities, viable damaged cells remain that can attack host tissue. These cells will seek out injury sites inside the body that are present due to physical damage, the activities of pathogenic organisms, parasites, chemical agents, or irradiation and establish colonies at those locations. The final morphology of the cancer cells develops as a result of interactions with the surrounding host tissue.

It is further hypothesized that secretions of the thyroid and adrenal glands and the pancreas play a critical role in the activation of a killer cell capable of eliminating cancer "progenitor" cells as well as established tumors.

The described natural formulas are intended to optimize the functioning of the pituitary gland as well as the other three endocrine glands leading to the restoration of normal immune functions. The effect of the formulas results in the regression of cancerous growth without the severe and sometimes fatal side effects of contemporary therapies.

A potential causative agent for cancer, besides the environmental factors, is prolonged periods of stress. Stress has been found to be associated with immunosuppression and an increased frequency of tumors (Lissoni, P. et al. Neuroendocrinol. Lett. (2001) 22: 175-180). Therefore, it is important that life-style changes also accompany any therapeutic Intervention if long-term results are sought.

The following compositions according to claims 1-7 are described in the subject invention.

The therapeutic compositions described in the present application are summarized in the following mixtures of plant-derived and mineral substances.

The first of these mixtures of plant-derived and mineral substances is called MSQ-11. More specifically, the active ingredients are blackstrap molasses, apple cider vinegar, quinine, and sulfur. The optimal composition is made up as follows:

Thoroughly mix in a blender the following Ingredients in this order:

1. 755 ml blackstrap molasses,
2. 59 ml apple cinder vinegar,
3. 3 g quinine (USP grade),
4. 29 g of sulfur (USP grade).

Blend on high speed, and add sufficient whole milk to bring the final volume to 1 quart (946 ml). The mixture is stored refrigerated. For long-term storage, it must be kept in a freezer. Additional optional ingredients are Vitamin B₁₂ (63 mg), folic acid (250 mg), and rose petal extract/rose oil (50 µl) dissolved in the final quart. One or more of these optional ingredients can be used. The formula including Vitamin B₁₂ and rose oil is designated MSQ-11A. For milk-intolerant patients, such as patients with lactose intolerance, the milk in the formula can be replaced by purified water.

In this first embodiment, to treat malignancies of the lymphoid system, the formula is complemented with the following additions and called MSQ-12.

1. 1/2 tsp of ground red pepper,
2. 2 tbsp of corn oil,
3. 177 ml of fresh squeezed pineapple juice,
4. 87g of finely ground raw almonds, and
5. 2 aspirins a day taken separately by the patient.

The MSQ-11 formula can be used on its own (without Soma) if iodine (USP 23, Strong Iodine Solution) is added to the composition in the amount of 6-9 ml per 1quart (946 ml) final volume that is equivalent to 4 -6 drops per 30 ml single dose. This formula, including the iodine, is called MSQ-13.

In another alternative, the composition is designated MSQ-14. The composition of MSQ-14 formula is as follows: 755 ml of blackstrap molasses, 59 ml apple cider vinegar, 89 ml blackberry juice, 29 ml distilled water, 8 drops of USP 23 Strong Iodine Solution, 3.7 ml rose oil, 29 g sulfur, 3 g of quinine sulfate, 59 g baking soda in small portions, 59 g of vitamin C calcium salt, and, optionally, 1.5 g of a carbonaceous material selected from the group consisting of charcoal dust and diamond dust. The daily dose of MSQ-14 is 3x2 1/2 tbsp.

In yet another alternative, the composition is designated MSQ-15. The MSQ-15 formula is an improvement over the MSQ-13 by adding 30 ml of prune juice and 21 g of powdered sugar (sucrose) as accelerators to every quart of formula.

In still another alternative, the composition is designated MSQ-16. The MSQ-16 formula is an improvement over the MSQ-15 by adding an additional 1.2 g of cayenne pepper and 44 g of baking soda to every quart.

In this first embodiment, the composite mixture (i.e., one of MSQ-11, MSQ-11A, MSQ-12, MSQ-13, MSQ-14, MSQ-15, or MSQ-16) is administered orally at a dose of 1-3 tbsp (15-45 ml) for adults, preferably at 2 tbsp, three times a day taken with meals. Along with this treatment, 6 glasses of water should be taken daily spaced at proper intervals. This administration schedule is followed for 3-4 weeks. The administration of this therapy may continue depending on the rate of cancer regression. The therapy can be used prophylactically after remission is obtained. Also it can be used as a cancer preventative agent.

As used herein, the term "therapeutic effect against cancer" means any effect against cancer, including but not limited to symptomatic relief, improvement in subjective well-being, histological improvement such as reduction in tumor burden, reduction in stage or grade of the tumor, reduction of tissue damage associated with malignancy, or other biological, pathological, or histological effects.

In another embodiment of the composition, one or more of the compositions described above as MSQ-11, MSQ-11A, MSQ-12, MSQ-13, MSQ-14, MSQ-15, or MSQ-16 are administered. In this embodiment, the composite mixture (MSQ-11, MSQ-11A, MSQ-12, MSQ-13, MSQ-14, MSQ-15, or MSQ-16) is administered orally at a dose of 1-3 tbsp (15-45 ml) for adults, preferably at 2 tbsp, three times a day taken with meals. Along with this treatment, 6 glasses of water should be taken dally spaced at proper intervals. This administration schedule is followed for 3-4 weeks. The dosage can be adjusted as needed depending on the response of the patient. The administration of this therapy may continue depending on the rate of cancer regression. The therapy can be used prophylactically after remission is obtained.

An advantage of the present invention is a method of treating cancer comprising administering a composition or compositions according to the present invention to a patient in need thereof.
In particular the composition can be one of MSQ-11, MSQ-11A, MSQ-12, MSQ-13, MSQ-14, MSQ-15, or MSQ-16. If the composition is a composition according to the present invention including ground red pepper, com oil, pineapple juice, and raw almonds, the method preferably further comprises administering aspirin to the patient.

The malignancy to be treated can be, but is not necessarily limited to, a malignancy of the lymphoid system. The malignancy can be another malignancy, such as pulmonary squamous cell carcinoma or squamous cell carcinoma of the cervix, as well as other types of solid tumors.

The following Examples illustrate the advantages of the subject invention. These Examples are illustrative only and are not intended to limit the invention. Accordingly, it is to be understood that the description in this disclosure is to facilitate comprehension of the invention and should not be construed to limit the scope thereof as persons skilled in the art can, in light of this disclosure, generate additional embodiments without exceeding the scope or departing from the spirit of the claimed invention.

EXAMPLE 1

### Resolution of Pleuritis Carcinomatosa and Atelectasis in an Inoperable Malignant Lung Cancer

A 55-year-old male patient was admitted to the hospital on 10-27-1999 with right-sided chest pain, hemoptysis, worsening shortness of breath, and dyspnea on exertion.

He had a history of smoking for 40 years, 1.5-2 packs a day. He claimed that he consumed 1 glass of wine and 2 bottles of beer a day. He suffered myocardial infarction in May 1999. On physical exam he was an obese man who appeared older than his chronological age. Chest and throat exam revealed emphysema and severe chronic laryngitis. Cardiac enlargement and hepatomegaly was observed. Extremities were free of edema and clubbing.

His blood gases on room air were pO₂ 61.9, pCO₂ 52.6, pH 7.39 and Sat **91%.** A CT of the chest revealed a large mass in the third segment of the right lung that propagated onto the pleura. Around the mass, distelectasis and infiltration was observed. Pleural fluid or abnormal lymph nodes were absent.

A biopsy was performed and the initial finding was a partially undifferentiated squamous cell carcinoma. Due to the patient's cardio-respiratory status and the extent of the infiltration, the tumor was evaluated as inoperable. The exact size of the tumor could not be determined.

He was placed on a Carboplatin and Vepesid^{™} combination chemotherapy and radiation therapy. He received 2 cycles of chemotherapy and 30 Gy of irradiation. A chest CT taken on 03-03-2000 had shown the presence of a 5 cm diameter tumor in the upper right lobe that contained an irregular internal cavity, and showed propagation onto the pleura. In April 2000, the patient decided to discontinue the therapies due to their severe side effects.

Starting in June 2000, the patient has taken a one month long course of the oral combination herbal supplement, MSQ-11. The active ingredients are molasses, apple cider vinegar, quinine and sulfur. The dosage was 3 x 1 tablespoons a day taken with meals until 1 quart of the mixture was consumed. Ample consumption of whole milk with the formula was recommended.

Two weeks after the initiation of MSQ-11 supplementation, hemoptysis resolved. Shortly after completing the course, pneumonia developed specifically affecting the tumor site. Antibiotics were prescribed and the pneumonia subsequently resolved.

After the resolution of the pneumonia, the patient enjoyed a relatively uneventful three months before he was again admitted to the hospital on 10-13-2000 with right-sided anterior chest pain, shortness of breath, dyspnea on exertion and peripheral edema.

His blood gases were PO₂ 5.59 kPa, pCO₂ 7.60 kPa, pH 7.383 and Sat 79.8%. A chest X-ray revealed the progression of the tumor in the upper right lobe as well as pleural fluid accumulation. Complete atelectasis of the right lung had developed. During pleurocentesis, 650 ml of fluid was removed which contained blood, large numbers of lymphocytes, macrophages and mesothelial cells. In the cytology report, there is no mention of tumor cells. Subsequently, he was released and instructed to return in the event if his dyspnea was worsening.

Five weeks later on 11-20-2000, the patient was readmitted to the hospital with fever (37.7-38.8°C), shortness of breath, and dyspnea on rest. His blood gases on admission were PO₂ 43, pCO₂ 52, pH 7.43 and Sat 79%. Chest x-ray revealed the progression of the upper right lobe tumor with an expansion into the central lobe. Complete atelectasis of the right lung and hydrothorax had developed. During another pleurocentesis, 800 ml of pleural fluid was removed. The pleural fluid contained large numbers of white blood cells. The general condition of the patient did not allow chemotherapy. At this point, the prognosis was extremely bleak.

At this time, a combination of Soma extract being not a part of the present invention and MSQ-11 was given. One week after his second pleural drainage, the patient started taking 1 ml of Soma extract twice a day, dissolved in a cup of water (starting on 11-29-2000). Soma was administered for six weeks. At three weeks into the Soma (not belonging to the present invention) therapy; MSQ-11 was added to the regimen at 3 tbsp per day for one month. A maintenance dose of 1 tbsp of MSQ-11 a day was used for an additional month after completing this standard treatment course.

The patient's hypoxia was relieved with a nutritional supplement, called Aerobic 07 (Aerobic Life Industries, Phoenix, AZ, USA). Aerobic 07 delivers oxygen directly into the circulation via the stomach. The dosage was 10 drops dispersed into a cup of water, taken twice a day, following the general recommendations of the manufacturer. The patient reported an immediate relief from his dyspnea upon taking the first dose of Aerobic 07. Four days later upon his discharge, the patient's blood oxygen saturation was 88%.

He continued using Aerobic 07 at the same dose for 4 months and for another 4 months at a half dose. The patient reported Aerobic 07 to be very important in improving his general well being. Repeated determination of oxygen saturation has shown a continuous progress.

On 01-03-2001, the patient was readmitted to the hospital, and 350 ml of yellow pleural fluid was removed. Cytology found a few lymphocytes and macrophages with no blood or tumor cells present. This time, it appeared that his pleuritis carcinomatosa was subsiding. Blood gases were pO₂ 7.06 kPa, pCO₂ 6.40 kPa, pH 7.462 and Sat 89.1%.

One week later on 01-10-2001, ahead and chest CT was performed. No abnormalities were found inside the cranium. Abnormal lymph nodes were absent in the mediastinum. A circular constriction of the upper right lobar bronchus was observed. In the upper right lobe, tumorous infiltration was apparent. The size of the upper right lobe tumor could not be determined. The image suggested necrosis. Pleural fluid accumulation was noted but it was insufficient for tapping. He was released from the hospital.

From January the patient lived at home and reported a relatively good quality of life. On 03-26-2001, the patient checked into the hospital because of chest pain. His blood gases were pO₂ 8.73kPa, pCO₂ 5.89kPa, pH 7.421 and Sat 93.1%. The patient's blood oxygen saturation had returned to normal. Chest x-ray found no tumor progression and pleural fluid was undetectable. The episode was diagnosed as viral infection.

Four months later, he was admitted to the hospital again because of right-sided chest pain. Chest x-ray has shown no change since his previous admission. An ultrasound exam found no abnormalities in the organs inside the abdomen. Results of any cardiac evaluation could not be found. The chest pain was attributed to scar tissue formation in the upper right lobe. He was given pain medication and released.

A month later, the patient was admitted to the hospital, this time with worsening signs of congestive heart failure. At the same time, chest x-ray showed progression of the upper right lobe tumor. Pleural fluid was undetectable. MSQ-11 administration was initiated. His cardiac functions continued to deteriorate and were not responding to therapy. Three weeks later, he deceased. At the family's request, no autopsy was performed.

DISCUSSION

Lung cancer can be linked to tobacco smoking in the majority of cases (Doll, R, Gray R, Hafner B, and Peto R. Br Med J 280: 961-971, 1980; Doll R and Hill A. Br Med J 2: 1071-1081, 1956). Despite the great expansion of understanding cancer biology, lung cancer remains one of the deadliest human neoplasias. About 90% of lung cancer patients die due to the worst cure rates among common solid tumors ("Cancer Facts and Figures2000." 2000 American Cancer Society, Atlanta). New therapeutic strategies are therefore needed that can improve current prospects for long-term survival from lung cancer.

In this Example, we presented a patient's case with rapidly progressing, large, partially undifferentiated squamous cell carcinoma (Marchevsky AM. Malignant epithelial tumors of the lung. In: Marchevsky AM, ed. Surgical pathology of lung neoplasms. New York, Marcel Dekker, 1990:77-229) and demonstrated the resolution of carcinomatous pleuritis and atelectasis developed during the progression of his carcinoma. The patient had a history of emphysema and acute myocardial infarction. His critical condition and the bleak prognosis of his disease qualified him for this alternative approach.

The progression of the tumor discontinued and the pleuritis carcinomatosa resolved over a period of 2 months while using a combination of Soma and MSQ-11. The patient reported a gradual increase in his energy and an overall improvement in the quality of his life that lasted for nearly 8 months. He experienced no side effects during Soma (not belonging to the present invention) and MSQ-11 administration. Additional oral oxygenation was effective in relieving the patient's dyspnea and improved oxygenation may have contributed to the overall effects of Soma and MSQ-11.

This study described how the administration of a combination of natural remedies coincided with the regression of an originally inoperable lung carcinoma. We believe that this therapeutic modality will have a similar beneficial effect for all cancers including carcinomas, sarcomas and lymphomas.

EXAMPLE 2

### Tumor Regression in a Recurrent, Metastatic Squamous Cell Carcinoma of the Cervix: Case Report

Cervical carcinoma is a common gynecological neoplasia that caused 4,100 deaths in 2002 in the United States (Cancer Facts and Figures-2002." American Cancer Society, Atlanta 2002).

Radical pelvic surgery and radiation therapy is the mainstay in treatment (Thar T, Milton RR, Daily JW: Radiation treatment of the carcinoma of the cervix. Semin Oncol 9; 299-311, 1982).

Chemotherapy is generally reserved for treatment of locally recurrent disease (DeVita VT, Wasserman TH, Young RC: Perspectives in research in gynecologic oncology. Cancer 38; 509-525, 1991 and Alberts DS, Garcia D, Mason-Liddil N: Cysplatin in advanced cancer of the cervix: An update. Semin Oncol 18; 11-24, 1991).

Despite advances in surgical techniques, radiation, and chemotherapy, stage-specific survival rates of patients with locally advanced cervical cancer have not improved (Thar T, Milton RR, Daily JW: Radiation treatment of the carcinoma of the cervix. Semin Oncol 9; 299-311, 1982 and Petterson F (ed.): Carcinoma of the uterine cervix. In: Annual report on the results of treatment in gynecological cancer. FIGO 22; 51-63, 1994). Therefore, it is important to develop new treatment modalities in order to improve current prospects for long-term survival.

In this report, a patient's case is presented with a recurrent, metastatic squamous cell carcinoma of the cervix. The report demonstrates that a novel, nutritional combination therapy produced tumor regression and no evidence of disease.

Methods

In June 1998, a 43 year-old patient was diagnosed with squamous cell carcinoma of the cervix (grade II/A) and metastasis to the colon. She underwent Wertheim's radical hysterectomy and pre- and post-operative radiation therapy for a total dose of 50 Gy.

In June 2000, she was diagnosed with right-sided hydronephrosis, rectovaginal fistula, and recurrent malignant disease in the pelvis. Rectovaginal exam confirmed a 6-cm tumor that was partially attached to the pelvic wall and infiltrated the base of the bladder as well as the colon. In July 2000, 6 cycles of the Cysplatin-Vepesid-Epirubicin combination chemotherapy was initiated. She received 2 cycles of chemotherapy (one each in July and August). Because of the serious side effects of the treatment (myelosuppression, nausea, vomiting and severe pain in the flanks and extremities) no further chemotherapy was administered and the patient received only red blood cell transfusions and pain medication from that on. Her prognosis was poor.

Starting in the beginning of November 2000, the patient has taken a one month long course of MSQ-11. This formula was established based on the analysis of the scientific literature on the effects of nutrition on a variety of cancers The active ingredients are blackstrap molasses, apple cider vinegar, quinine and sulfur. The dosage was 1 tbsp TID po taken with meals until 1 quart (946 ml) of the mixture was consumed, then 1 tbsp QD for another 5 months. Ample consumption of whole milk or purified water with the formula is recommended.

The patient was monitored periodically by clinical examinations and by May 2001 no tumor could be detected clinically or otherwise. In September 2001, the patient underwent an ileus surgery and the preparation of a temporary preternatural anus.

The patient remained stable until January 2002, when she presented with high fever and right flank pain. This was attributed to a urinary infection caused by the yet unresolved rectovaginal fistula. Abdominal ultrasound exam has shown a right-sided renal abscess, the resolution of which required right-sided nephrectomy. In March 2002, an abdominal CT exam has shown an irregularly shaped growth in the right side of the pelvis that has accumulated contrast material and was in contact with the base of the bladder as well as the adjacent intestines.

A gynecological exam in April 2002 has found a mass around the vaginal cuff that filled the entire pelvis area and was suspected to be a recurrent tumor. Pathological exam has shown the recurrence of SCC of the cervix (grade III-IV). The tumor was evaluated to be inoperable. In the same month, the surgical repair of the rectovaginal fistula was carried out. At this point, she has resumed taking MSQ-11 and Soma. Soma was administered at the same dosage as in 2001 while the dosage of MSQ-11 was 2tbsp TID. The tumor regression was monitored by pelvic ultrasound, and clinical examinations. Subsequently, the nutritional supplement regime was adapted according to the clinical status and ultrasound results.

In May 2002, an ultrasound exam has shown a highly vascularized, 41x53x60mm size tumor in the right side of the pelvis that has infiltrated the wall of the bladder. Thus, a more active formula, MSQ-11A that also contained vitamin B₁₂ and rose oil was administered instead of MSQ-11 at 2 tbsp TID. The use of Soma was discontinued.

In June 2002, an ultrasound exam has shown a 40x27x25x28mm size tumor in the right side of the pelvis that was attached to the bladder over an area of 2cm diameter and to the colon over an area of 1.7cm diameter. A subsequent CT exam has shown the adhesion of intestinal loops to the base and the right side of the bladder. Inside the conglomerate, a 3cm diameter solid formation was found that was thought to be the tumor.

An ultrasound exam in July 2002, has demonstrated a 30x33x60 mm size irregular-shaped formation in the right side of the pelvis. To potentially accelerate tumor regression, MSQ-11A was replaced with the more active MSQ-13 formula. MSQ-13 contains folic acid and molecular iodine as additional components. With MSQ-13 administration Soma was discontinued. After completing the basic course of MSQ-13 therapy (2 quarts), a pelvic hemorrhagic episode occurred. Large blood clots were spontaneously discharged rectally and vaginally. The surgical team interpreted this as possible healing process. The anemia caused by the hemorrhage resolved without a need for intervention. The patient continued with the nutritional supplementation. Subsequent ultrasound exam has found an 8 x 5 cm formation in the right side of the pelvis. The enlargement was probably caused by the hemorrhage.

A CT exam in late September 2000 has found an irregular-shaped soft tissue conglomerate in the right side of the pelvis that was attached to the right side of the bladder. Above the vaginal fornix, a 4-5 cm-size solid formation was found. The oral contrast material accumulated inside the vagina (vaginal tampon) indicating the presence of a still existing fistula. No accumulation of contrast material was observed elsewhere. Abnormal lymph nodes were absent in the retroperitoneum. An MRI exam was prescribed to differentiate the tumor from the surrounding scar tissue.

Subsequent to the hemorrhage, the patient noticed a gradually intensifying inflammation in her pelvis by early October 2002. On multiple occasions, spontaneous putrid smelling vaginal and rectal discharges occurred that were accompanied with episodes of fever (37-38°C). After a gynecological exam and a CT scan in November 2002, she was referred to emergency surgery during which an about 12 cm size abscess was drained in the lower abdomen. The examination of the surrounding area indicated a potential continuation of the abscess. A lower pelvic laparotomy was performed that opened another, larger abscess that stretched from behind the symphysis towards the vaginal cuff. Following the lysis of small bowel adhesions, an encapsulated 3.5 x 5 cm size tumor, extensively attached to the sacrum, was observed. No metastases were found in the abdominal cavity.

The small bowel fistula could not be repaired at this time and the patient was scheduled for another surgery in February 2003. The fistula repair was attempted at the end of February 2003; however it turned out to be unsuccessful. Another surgery was performed in the middle of April 2003 to close the fistula but, again, it was unsuccessful. The patient deceased at the end of April 2003. No autopsy was performed at the family's request.

Discussion

Survival for women with cervical cancer has improved over the years primarily because of earthy diagnosis. For advanced disease, the 5-year survival rates remain unchanged (Thar T, Milton RR, Daily JW: Radiation treatment of the carcinoma of the cervix. Semin Oncol 9; 299-311, 1982 and Petterson F (ed.): Carcinoma of the uterine cervix. In: Annual report on the results of treatment in gynecological cancer. FIGO 22; 51-63, 1994). The 31% survival for stage III and 8% for stage IVA disease indicates that radiotherapy is not an effective therapeutic modality. Chemotherapy has been used for the management of locally recurrent disease but objective and subjective responses are of short duration (Alberts DS, Garcia D, Mason-Liddil N: Cysplatin in advanced cancer of the cervix: An update. Semin Oncol 18; 11-24, 1991 and Petterson F (ed.): Carcinoma of the uterine cervix. In: Annual report on the results of treatment in gynecological cancer. FIGO 22; 51-63, 1994). The prognosis for recurrent, locally advanced cervical carcinoma is poor.

This case study demonstrates that a combination of nutritional supplements may offer an effective tool for the management of recurrent SCC of the cervix. This patient's critical condition and her poor prognosis qualified her for this alternative approach.

This paper describes a non-toxic, nutritional therapy that was established based on the analysis of the scientific literature on the effects of nutrition on a variety of cancers. This analysis led to the conclusion that nutrition can provide a unifying perception of cancer and recast it as a single disease potentially treatable by a single protocol.

Nutritional deficiencies of plant-derived phenolic compounds, lipids, vitamins and minerals have been identified in a variety of cancers. Based on these, it was hypothesized that the supplementation of these nutrients of adequate amounts to cancer patients might reverse the course of the disease as human cells do have the capacity of self-repair.

Her general condition was very weak in the beginning of nutritional supplementation. Shortly after the initiation of supplementation, her appetite returned and over a time period of 2 months, she has regained all her lost weight. Myelosuppression and neuropathy have resolved. Her general condition improved such that she has resumed her daily routines. Clinical data confirmed tumor regression and subsequently has shown no evidence of disease. She has experienced no side effects during administration of nutritional supplementation.

The unresolved rectovaginal fistula, a frequent combined side effect of disease and intensive pelvic radiation therapy, led to serious complications. A renal abscess developed, the resolution of which required right-sided nephrectomy. A satisfactory recovery allowed the subsequent repair of the rectovaginal fistula. Pathological exam confirmed a recurrence of SCC of the cervix, which this time has infiltrated the colon and the bladder.

The patient has resumed taking Soma (not belonging to the present invention) and MSQ-11 that later on was replaced with the more active MSQ-11A and MSQ-13. Tumor regression of greater than 50% and the resolution of metastases were observed during the course of her second alternative treatment. Her general condition was fair. The patient has undergone three surgeries in 2002 and two more in 2003 in an attempt to repair the active fistulas. Unfortunately, the conditions of her bowels have made the repair attempts unsuccessful and the patient deceased despite all the improvements in her oncological status.

Further studies are warranted to investigate the utility of this nutritional approach in a larger population of cervical cancer patients.

EXAMPLE 3

### Complete Remission in Acute Myelogenous Leukemia Achieved by a Nutrition-Based Therapy: Case Report

Introduction

Acute myelogeneous leukemia (AML) is a common form of adult leukemia accounting for 25% of all cases of leukemia (Hillman RS, Ault KA (eds): The acute myeloid leukemias. In: Hematology in clinical practice. McGraw-Hill, New York 1998). AML involves malignant transformation of myeloid cells inside the bone marrow leading to anemia, platelet deficiency and elevated white cell counts. The treatment outcome for adult AML remains poor (Cripe LD, Hinton S: Acute myeloid leukemia in adults. Current Treat Options Oncol 1; 9-17, 2000). High-dose chemotherapy is the mainstay of current therapeutic regimes, however it is highly toxic and may become fatal. In a recent clinical study, nearly one-third of the patients died during induction chemotherapy (Kakepoto GN, Bumey IA, Zaki S, Adil SN, Khurshid M: Long-term outcomes of acute myeloid leukemia in adults in Pakistan. J Pak Med Assoc 52; 482-486, 2002). Relapse is common among those who achieve complete remission from chemotherapy (Cripe LD, Hinton S: Acute myeloid leukemia in adults. Current Treat Options Oncol 1; 9-17, 2000). The long-term (2-4 years) survival without intensive post-remission therapies remains low, 2% to 10%, depending on the age groups (Kakepoto GN, Burney IA, Zaki S, Adil SN, Khurshid M: Long-term outcomes of acute myeloid leukemia in adults in Pakistan. J Pak Med Assoc 52; 482-486, 2002, and Menzin J, Lang K, Earle CC, Kemey D, Mallick R: The outcomes and costs of acute myeloid leukemia among the elderly. Arch Intern Med 162; 1597-1603, 2002). It is important to develop new, non-toxic treatment modalities that might offer a longer disease-free survival, particularly for the elderly. It has been found that a nutritional combination therapy described in this Example produced complete remission of AML.

Case report

In October 1999, a 28 year-old female patient sustained severe injuries at her lower extremities in an automobile accident after which she underwent reconstructive surgery and was hospitalized for three weeks. In December 1999, she underwent surgery for removal of metallic objects from her legs that were followed by a knee arthroscopic procedure in January 2000. In August 2001, she was diagnosed with a brain abscess that was drained surgically and subsequently treated with i.v. antibiotics. In January 2002, she was treated for infectious mononucleosis. In March 2002, she was diagnosed with polytoxicomania (benzodiazepine and synthetic opiate dependency) and subsequently treated for withdrawal symptoms.

In May 2002, the patient was diagnosed with cervical intraepithelial neoplasia and subsequently treated with conization and cryocoagulation. She also received cytostatic (Endoxan, Cysplatyl, Vinblastin, Cosmegen), antihormone (Zitazonium, Honvan) and interferon (Intron A, Egiferon) therapies. She experienced the usual side effects of chemotherapy (nausea, vomiting and severe pain in the extremities).

The patient also reported alternating episodes of diarrhea and constipation. She has lost weight, felt pain in the bones and was fatigued most of the time. Hemoptysis and menorrhagia was noted.

In July 2002, the patient was treated for acute pancreatitis. New diagnosis indicated thrombocytopenia, elevated white cell counts and pernicious anemia. In September 2002, a growth appeared on the upper arm. Pathological exam confirmed an unclassified soft tissue malignancy and the growth was surgically removed. Subsequently, nodules appeared in the armpits, on the neck and around the genitalla. At the beginning of October 2003, a whole body CT scan was performed.

Numerous (the exact numbers could not be determined) around 1 cm diameter soft tissue tumors were detected throughout the body. The highest density of the tumors was in the lower abdomen, underneath the right side of the ribcage as well as the right side of the spine. Significant fluid accumulation was detected in the pelvic area.

Starting at the end of October 2003, the patient has taken a one month long course of MSQ-13 oral nutritional supplement. This nutritional supplement was developed as a result of the discovery that a variety of common nutritional deficiencies are present in cancer patients. The active ingredients of the supplement are blackstrap molasses, apple cider vinegar, quinine, sulfur, rose oil, folic acid, vitamin B₁₂ and molecular iodine. The recommended dosage was 2 tbsp TID p.o. taken with meals. Ample consumption of whole milk or purified water with this formula is necessary.

At that point, the patient had difficulties to swallow and could only take half the recommended dose of MSQ. Despite this, some improvement in the blood work was observed by 11/19/2003 (Table 1) and some regression of visible tumors located along the spine was observed. At the end of November 2003, an abrupt leukemic crisis occurred. The patient's RBC and platelet counts sharply declined while a surge in white cell counts was observed (Table 1). Bone marrow pathology confirmed the presence of erythroid blast cells in the bone marrow that were also detectable in the peripheral blood.

For the next month, she could only sporadically take MSQ. Her symptoms now also included vomiting, constant fever (>38°C), episodes of alternating high and low blood pressure, elevated pulse rate, sweating, shortness of breath, bleeding and periodic loss of consciousness. She has received parenteral nutrition as well as vitamin and mineral supplementation. In the middle of January 2003, the blast cell concentration in the marrow was 54% and the patient also presented bacteriuria (>100,000 cfu/ml) and proteinuria (>500 mg/24 h).

Oral feeding resumed with the aid of a fruit concentrate-based nutrient. By the end of January, 2003 she has also resumed taking MSQ. This time she has received MSQ-15, an improvement over MSQ-13. MSQ-15 contains additional prune juice and sucrose acting as accelerators.

By 02/24/03, there was a surge in RBC and platelet counts and a decline in white blood cells (Table 1). Blood enzymes and metabolites also returned into their normal ranges. Blast cells disappeared from the bone marrow and the peripheral blood, all the soft tissue tumors regressed and the pathological urinary symptoms reversed. All the physical symptoms of AML have resolved; she had a complete remission.

Discussion

AML is a myeloproliferative disorder that typically affects the elderly but it can be present at any age. The majority of adults relapse after undergoing highly toxic chemotherapeutic regimes that can be lethal to a relatively large proportion of patients (Kakepoto GN, Bumey IA, Zaki S, Adil SN, Khurshid M: Long-term outcomes of acute myeloid leukemia in adults in Pakistan. J Pak Med Assoc 52; 482-4861 2002). Long-term survival for adults remains poor (Cripe LD, Hinton S: Acute myeloid leukemia in adults. Current Treat Options Oncol 1; 9-17, 2000, Kakepoto GN, Bumey IA, Zaki S, Adil SN, Khurshid M: Long-term outcomes of acute myeloid leukemia in adults in Pakistan. J Pak Med Assoc 52; 482-486, 2002, and Menzin J, Lang K, Earle CC, Kemey D, Mallick R: The outcomes and costs of acute myeloid leukemia among the elderly. Arch Intern Med 162; 1597-1603, 2002). This paper describes a non-toxic, nutrition based therapy that was established based on the analysis of the scientific literature on the effects of nutrition on a variety of cancers. This analysis led to the conclusion that nutrition can provide a unifying perception of cancer and recast it as a single disease potentially treatable by a single protocol.

Nutritional deficiencies of plant-derived phenolic compounds, folate, vitamin B₁₂ as well as other vitamins of the vitamin B class, essential lipids, iodine, and several minerals have been found to co-exist in and lead to a variety of cancers. Based on these, it was hypothesized that the supplementation of these nutrients of adequate amounts to cancer patients might reverse the course of the disease as human cells do have the capacity of self-repair. This case study demonstrates the result of this hypothesis on an adult female AML patient.

The administration of the MSQ-15 nutritional supplement cocktail to this patient led to a rapid reversal of the characteristic cellular abnormalities of AML both in the bone marrow and peripheral blood along with the complete regression of accompanying disseminated soft tissue tumors. Based on these objective criteria, a complete remission was obtained without the toxic side effects and potential lethality of induction chemotherapy.

This case study demonstrates that a combination of nutritional supplements may offer an effective tool for the management of AML. Further studies are warranted to Investigate the utility of this approach in a larger population of AML patients.

## Claims

1. A therapeutic composition, comprising a mixture of plant derivatives and a mineral comprising:
(a) apple cider vinegar;
(b) quinine;
(c) blackstrap molasses;
(d) sulf ur; and
(e) an ingredie nt selected from the group consisting of whole milk and water; wherein the apple cider vinegar, the quinine, the blackstrap molasses, and the sulfur are each present in a sufficient quantity so that the resulting composition has a therapeutic effect against cancer.

2. The composition according to claim 1, wherein the composition comprises water.

3. The composition according to claim 1, wherein the composition comprises whole milk.

4. The composition according to claim 3, wherein the mixture of plant derivatives and a mineral comprises about 6.23% (v/v) of apple cider vinegar, about 0.317% (w/v) of quinine, about 79.8% (v/v) of blackstrap molasses, and about 3.1 (w/v) of sulfur.

5. The composition according to claim 2, wherein the composition further comprises blackberry juice, iodine, rose oil, baking soda, vitamin C calcium salt, and, optionally, a carbonaceous substance selected from the group consisting of charcoal dust and diamond dust; wherein, the blackberry juice, the iodine, the rose oil, the sulfur, the quinine sulfate, the baking soda, and the vitamin C calcium salt are each present in a sufficient quantity so that the resulting composition has a therapeutic effect against cancer.

6. The composition of any of claims 1 to 5 for use as an active pharmaceutical substance.

7. Use of the composition of any of claims 1 to 6 for the preparation of a pharmaceutical composition for the treatment of cancer.

## Patentansprüche

1. Therapeutische Zusammensetzung, umfassend eine Mischung aus Pflanzenderivaten und einem Mineral, umfassend:
(a) Apfelessig
(b) Chinin;
(c) Restmelasse;
(d) Schwefel; und
(e) einen Inhaltsstoff ausgewählt aus der Gruppe bestehend aus Vollmilch und Wasser, wobei der Apfelessig, das Chinin, die Restmelasse und der Schwefel jeweils in einer ausreichenden Menge vorhanden sind, so dass die erhaltende Zusammensetzung eine therapeutische Wirkung gegen Krebs hat.

2. Zusammensetzung nach Anspruch 1, wobei die Zusammensetzung Wasser umfasst.

3. Zusammensetzung nach Anspruch 1, wobei die Zusammensetzung Vollmilch umfasst.

4. Zusammensetzung nach Anspruch 3, wobei die Mischung aus Pflanzenderivaten und einem Mineral ungefähr 6,23% (v/v) Apfelessig, ungefähr 0,317% (w/v) Chinin, ungefähr 79,8% (v/v) Restmelasse und ungefähr 3,1% (w/v) Schwefel umfasst.

5. Zusammensetzung nach Anspruch 2, wobei die Zusammensetzung weiterhin Brombeersaft, Iod, Rosenöl, Natron, Vitamin C-Calciumsalz und gegebenenfalls eine kohlenstoffhaltige Substanz ausgewählt aus der Gruppe bestehend aus Kohlestaub und Diamantstaub umfasst; wobei der Brombeersaft, das Iod, das Rosenöl, der Schwefel, das Chininsulfat, das Natron und das Vitamin C-Calciumsalz jeweils in einer ausreichenden Menge vorhanden sind, so dass die erhaltene Zusammensetzung eine therapeutische Wirkung gegen Krebs hat.

6. Zusammensetzung nach einem der Ansprüche 1 bis 5 zur Verwendung als aktive pharmazeutische Substanz.

7. Verwendung der Zusammensetzung nach einem der Ansprüche 1 bis 6 zur Herstellung einer pharmazeutischen Zusammensetzung zur Behandlung von Krebs.

## Revendications

1. Composition thérapeutique, qui comprend un mélange de dérivés végétaux et d'un minéral, qui comprend :
(a) du vinaigre de cidre ;
(b) de la quinine ;
(c) de la mélasse de cuisine ;
(d) du soufre ; et
(e) un ingrédient choisi dans le groupe constitué par le lait entier et l'eau ;
le vinaigre de cidre, la quinine, la mélasse de cuisine, et le soufre étant chacun présents en une quantité suffisante pour que la composition résultante ait un effet thérapeutique contre le cancer.

2. Composition selon la revendication 1, dans laquelle la composition comprend de l'eau.

3. Composition selon la revendication 1, dans laquelle la composition comprend du lait entier.

4. Composition selon la revendication 3, dans laquelle le mélange de dérivés végétaux et d'un minéral comprend environ 6,23 % (v/v) de vinaigre de cidre, environ 0,317 % (p/v) de quinine, environ 79,8 % (v/v) de mélasse de cuisine, et environ 3,1 (p/v) de soufre.

5. Composition selon la revendication 2, dans laquelle la composition comprend en outre du jus de mûre, de l'iode, de l'essence de rose, de l'hydrogénocarbonate de sodium, du sel de calcium de vitamine C et, facultativement, une substance carbonée choisie dans le groupe constitué par la poussière de charbon et la poussière de diamant ; le jus de mûre, l'iode, l'essence de rose, le soufre, le sulfate de quinine, l'hydrogénocarbonate de sodium et le sel de calcium de vitamine C étant chacun présents en une quantité suffisante pour que la composition résultante ait un effet thérapeutique contre le cancer.

6. Composition selon l'une quelconque des revendications 1 à 5, destinée à être utilisée en tant que substance pharmaceutique active.

7. Utilisation de la composition selon l'une quelconque des revendications 1 à 6, pour la préparation d'une composition pharmaceutique destinée au traitement du cancer.
